# FASCICULE DE BREVET EUROPEEN

(11) **EP 3 256 155 B1**
(45) Date de publication et mention de la délivrance du brevet: **19.06.2019**
(21) Numéro de dépôt: 16703811.6
(22) Date de dépôt: 11.02.2016
(51) Int. Cl.: A61K 39/00, A61K 9/00, A61K 45/06, A61K 9/06, A61K 9/08, A61K 9/107, A61K 31/4184, A61K 31/4523, A61K 31/517, A61K 31/519, A61K 31/7048, A61P 17/00

(54) **COMPOSE DE LA FAMILLE DES AVERMECTINES POUR LE TRAITEMENT ET/OU LA PREVENTION DES FOLLICULITES**
VERBINDUNG AUS DER AVERMECTIN-FAMILIE ZUR BEHANDLUNG UND/ODER PRÄVENTION VON FOLLIKULITIS
COMPOUND FROM THE AVERMECTIN FAMILY FOR TREATING AND/OR PREVENTING FOLLICULITIS

(30) Priorité: 12.02.2015 FR 1551162
(43) Date de publication de la demande: 20.12.2017
(73) Titulaire: Galderma S.A., 6330 Cham (CH)
(72) Inventeur: KEROB, Delphine, 92927 La Defense Cedex (FR); GERBER, Peter Arne, 40225 Düsseldorf (DE)
(74) Mandataire: Nederlandsch Octrooibureau
(86) Numéro de dépôt international: PCT/EP2016/052885
(87) Numéro de publication internationale: WO 2016/128499

(56) Documents cités:
- US-A- 5 952 372
- US-A1- 2015 011 489
- DAMIAN DIONA ET AL: "Demodex infestation in a child with leukaemia: treatment with ivermectin and permethrin.", INTERNATIONAL JOURNAL OF DERMATOLOGY SEP 2003, vol. 42, no. 9, septembre 2003 (2003-09), pages 724-726, XP002751769, ISSN: 0011-9059
- COTLIAR JONATHAN ET AL: "Demodex folliculitis mimicking acute graft-vs-host disease.", JAMA DERMATOLOGY DEC 2013, vol. 149, no. 12, décembre 2013 (2013-12), pages 1407-1409, XP002751768, ISSN: 2168-6084
- STEIN LINDA ET AL: "Efficacy and safety of ivermectin 1% cream in treatment of papulopustular rosacea: results of two randomized, double-blind, vehicle-controlled pivotal studies", JOURNAL OF DRUGS IN DERMATOLOGY, STRATEGIC COMMUNICATION IN DERMATOLOGY, NEW YORK, NY, US, vol. 13, no. 3, 1 mars 2014 (2014-03-01), pages 316-323, XP009180110, ISSN: 1545-9616
- Jennifer Bragg, et al.: "Papulopustular drug eruption due to an epidermal growth factor receptor inhibitors, erlotinib and cetuximab", eScholarship.org Dermatology Online Journal, vol. 13, no. 1 2007, XP002751894, Extrait de l'Internet: URL:http://escholarship.org/uc/item/3426f0 9x [extrait le 2015-12-07]
- GERBER PETER A ET AL: "Density of Demodex folliculorum in patients receiving epidermal growth factor receptor inhibitors", DERMATOLOGY,, vol. 222, no. 2, 1 janvier 2011 (2011-01-01), pages 144-147, XP009187517,
- PATRIZI ANNALISA ET AL: "Rosaceiform eruption induced by erlotinib.", DERMATOLOGIC THERAPY OCT 2008, vol. 21 Suppl 2, octobre 2008 (2008-10), pages S43-S45, XP002751770, ISSN: 1529-8019
- SEGAERT S ET AL: "Skin toxicities of targeted therapies", EUROPEAN JOURNAL OF CANCER, PERGAMON, GB, vol. 45, 1 septembre 2009 (2009-09-01), pages 295-308, XP026627079, ISSN: 0959-8049, DOI: 10.1016/S0959-8049(09)70044-9 [extrait le 2009-09-01]
- LAING ET AL: "Eosinophilic pustular folliculitis induced by chemotherapy", JOURNAL OF THE AMERICAN ACADEMY OF DERMATOLOGY, MOSBY, INC, US, vol. 54, no. 4, 1 avril 2006 (2006-04-01), pages 729-730, XP005338839, ISSN: 0190-9622, DOI: 10.1016/J.JAAD.2005.10.048

## Description

La présente invention porte sur un composé de la famille des avermectines pour son utilisation dans le traitement et/ou la prévention des folliculites induites par des agents thérapeutiques anti-cancéreux et plus particulièrement des agents de thérapie ciblée.

### ARRIERE-PLAN TECHNIQUE

De très nombreux effets toxiques sont associés à l'utilisation d'agents thérapeutiques anticancéreux et notamment les agents de thérapie ciblée. Les effets toxiques associés à ces types de traitements sont le plus souvent observés au niveau cutané, ce sont notamment les éruptions acnéiformes, les xéroses, les paronychies, les alopécies ou encore les inflammations des muqueuses. Les éruptions acnéiformes, dont font partie les éruptions papulopustuleuses ou encore folliculites, sont les effets toxiques cutanés les plus souvent observés chez les patients subissant un traitement chimiothérapeutique et notamment une thérapie ciblée, avec une occurrence d'environ 80%. Les folliculites correspondent à des inflammations des follicules pileux cutanés, formant une papulo-pustule. On fait également référence aux folliculites sous le terme général d'éruptions acnéiformes bien que ces dernières se différencient nettement de l'acné par l'absence de comédons mais également leur possible localisation au niveau du cuir chevelu. Elles apparaissent très rapidement après le début du traitement et le plus souvent au niveau des zones séborrhéiques telles que le cuir chevelu, le visage, le cou, le haut du dos ou encore la poitrine, avant d'éventuellement se propager à d'autres zones cutanées. Ces dernières peuvent être plus ou moins sévères et peuvent également être accompagnées de prurit, de douleurs mais aussi être compliquées par des infections bactériennes notamment par les *Staphylococus aureus.* Ces folliculites font donc partie des effets toxiques cutanés les plus fréquemment observés chez les patients subissant un traitement anti-cancéreux et notamment soumis à des thérapies ciblées c'est-à-dire dirigées spécifiquement contre des cibles moléculaires supposées jouer un rôle dans la transformation néoplasmique de la cellule cancéreuse.

A titre d'exemple de thérapies ciblées, les agents inhibiteurs des récepteurs du facteur de croissance épidermique (EGFR), sont couramment utilisés pour traiter les tumeurs solides. Les agents ayant une activité anti-EGFR sont soit des anticorps monoclonaux soit des inhibiteurs de tyrosine kinase. En effet, l'activation des récepteurs du facteur de croissance épidermique active les voies de signalisation responsables de la prolifération et de la survie cellulaires. Ces récepteurs sont impliqués dans l'oncogenèse de nombreuses tumeurs solides, et sont notamment surexprimés par les cellules malignes.

Les récepteurs au facteur de croissance épidermique étant largement exprimés au sein de l'épiderme, l'utilisation d'agents anti-EGFR est souvent accompagnée de nombreux effets toxiques cutanés et notamment de folliculites, observées chez plus de 85%. Ces folliculites peuvent également être causées par d'autres agents thérapeutiques utilisés en thérapie ciblée, tels que les inhibiteurs sélectifs d'une protéine kinase activée par des agents mitogènes telles que les kinases MEK1 et/ou MEK2. Ces agents inhibiteurs de MEK ciblent la voie de signalisation MAPK/ERK qui est très souvent hyper active dans certains cancers. On utilise notamment ces inhibiteurs de MEK dans le traitement des mélanomes et des cancers colorectaux. L'apparition de ces éruptions papulopustuleuses est observée chez plus de 90% des patients traités à l'aide d'inhibiteurs de MEK.

Compte-tenu de l'occurrence très élevée de ces folliculites, la sévérité de certaines d'entre elles et de l'inconfort non seulement physique mais aussi psychologique du patient, il est parfois nécessaire de limiter les doses de ces agents anti-cancéreux et parfois même d'interrompre la thérapie, mettant ainsi en péril l'efficacité du traitement préconisé par le médecin et donc, la santé du patient.

C'est pourquoi il est nécessaire de trouver une solution permettant de traiter et/ou prévenir l'apparition et/ou l'aggravation des folliculites induites par des agents anti-cancéreux et plus particulièrement par ceux utilisés en thérapie ciblée.

Des solutions existent aujourd'hui, telles que l'utilisation de benzoyl peroxide, d'antibactériens oraux ou topiques tels que les tétracyclines, ou encore d'acides rétinoïques. En revanche, l'efficacité de ces traitements est anecdotique et n'a pas été démontrée de façon formelle. En outre des effets secondaires parfois non négligeables peuvent être observés avec l'utilisation de ces agents pour le traitement et/ou la prévention de ces folliculites. Ainsi, les rétinoïdes peuvent entraîner une sécheresse cutanée, des irritations, des érythèmes, une desquamation et des picotements ou brûlures pour les patients traités. L'utilisation de tels traitements nécessite donc également l'application de multiples agents hydratants, humectants et adoucissants pour soulager le patient. Les corticostéroïdes oraux et/ou locaux peuvent être utilisés dans les cas les plus sévères mais ces derniers risquent fortement d'interférer avec l'efficacité des agents anti-cancéreux. La prise d'antibiotiques tels que la tétracycline, l'érythromycine, la minocycline et la doxycycline pour traiter ces folliculites médicamenteuses est aussi préconisée mais leur faible solubilité lipidique nécessite une administration régulière et fréquente qui peut engendrer des phénomènes de résistance bactérienne et favoriser le développement de ces organismes résistants, ainsi que des problèmes d'intolérance chez le patient notamment une photosensibilité ou encore des troubles digestifs.

Des solutions à visée prophylactique sont également proposées dans l'art antérieur, mais leur efficacité n'est pas satisfaisante. L'utilisation de tétracyclines par voie orale et plus particulièrement de doxycycline une fois par jour est la solution prophylactique la plus souvent utilisée, mais celle-ci permet seulement de diminuer la sévérité des éruptions papulopustuleuses et non pas de prévenir leur occurrence.

Pour lutter contre l'aggravation des folliculites en présence de pathogènes cutanés, il existe certains moyens, notamment l'utilisation d'antibactériens chimiques ou d'antibiotiques tels que la pénicilline ou l'ampicilline qui sont les plus classiquement utilisés. Cependant, ces solutions présentent un certain nombre d'inconvénients car le *Staphylococcus aureus* devient rapidement résistant aux antibiotiques. Bien que la demande de brevet publiée FR2740039, décrit l'utilisation d'une substance choisie parmi les aldéhydes et les agents bi-fonctionnels, de préférence le glutaraldéhyde, pour inhiber la fixation sur les kératinocytes et cornéocytes de souches de pathogènes tels que *Staphylococcus aureus*, ces traitements sont en général coûteux et nocifs aussi bien pour la santé que pour l'environnement.

A la lumière de l'énoncé précédent il existe un besoin considérable de trouver une solution efficace permettant de traiter et/ou prévenir l'apparition des folliculites mais aussi leurs complications telles que des surinfections bactériennes. Plus particulièrement il est nécessaire de proposer une solution efficace pour traiter et/ou prévenir l'apparition des folliculites induites par des agents anti-cancéreux et plus précisément par des agents de thérapies ciblée tout en limitant les effets secondaires observés dans l'art antérieur.

### RESUME DE L'INVENTION

Les inventeurs proposent d'utiliser des composés de la famille des avermectines et de préférence l'ivermectine pour le traitement et/ou la prévention, de préférence par voie topique, des folliculites induites par des agents anti-cancéreux ayant une activité anti-EGFR ainsi que leur potentielle aggravation en présence de bactéries telles que les *Staphylococcus aureus.*

La présente invention porte plus généralement sur des composés de la famille des avermectines, pour leur utilisation dans le traitement et/ou la prévention des folliculites induites par des agents anti-cancéreux ayant une activité anti-EGFR. Elle porte également sur une composition pharmaceutique, de préférence destinée à une administration topique, comprenant le composé de la famille des avermectines, pour son utilisation dans le traitement et/ou la prévention des folliculites induites par des agents anti-cancéreux ayant une activité anti-EGFR. Dans un mode de réalisation préféré, le composé de la famille des avermectines est l'ivermectine.

La présente invention est définie dans les revendications annexées. Les modes de réalisation qui ne relèvent pas de ces revendications sont uniquement à des fins de référence.

### LEGENDE DES FIGURES

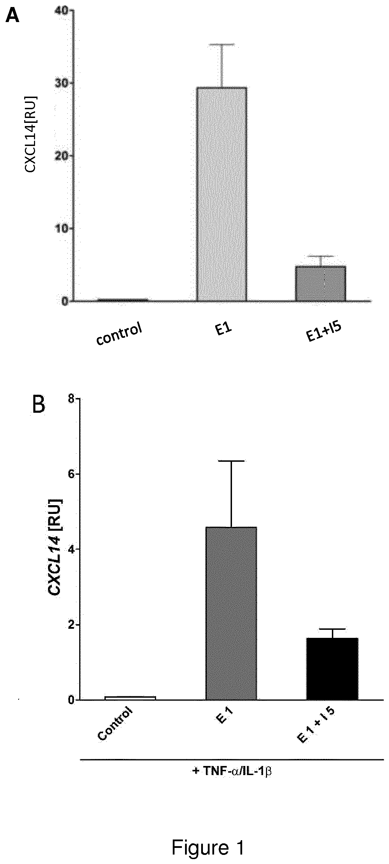

Figure 1 : Analyse de l'expression de CXCL14 par les kératinocytes traités avec l'erlotinib seul ou en combinaison avec l'ivermectine (A) ou avec l'erlotinib seul ou avec l'ivermectine, en présence de TNFα/IL1β (B). E1= erlotinib, 1µM ; I5= ivermectine, 5µM

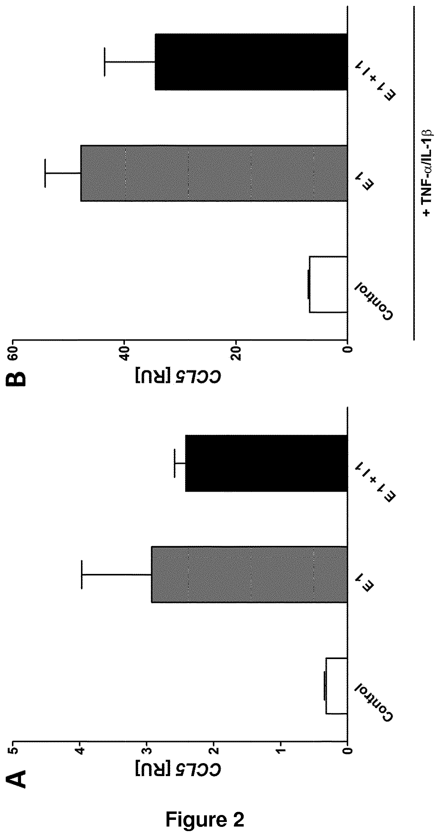

Figure 2 : Analyse de l'expression de CCL5 par les kératinocytes traités avec l'erlotinib seul ou en combinaison avec l'ivermectine (A) ou avec l'erlotinib seul ou avec l'ivermectine, en présence de TNFα/IL1β (B). E1= erlotinib, 1µM ; I1= ivermectine, 1µM

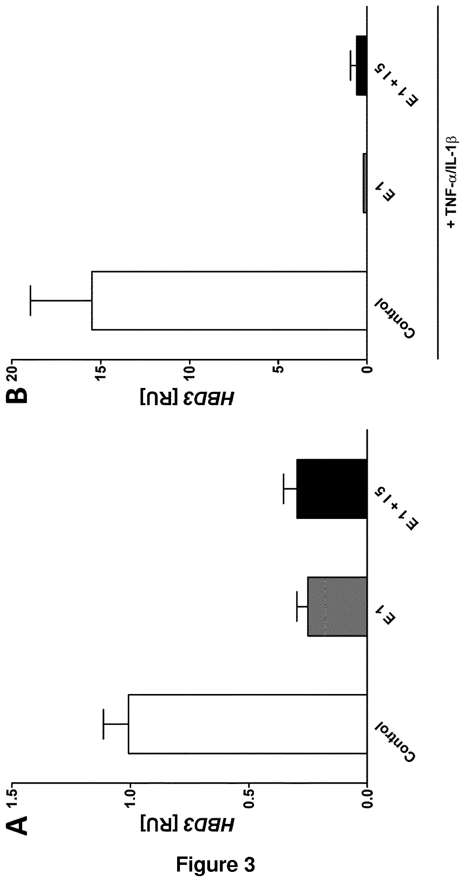

Figure 3: Analyse de l'expression de HBD3 par les kératinocytes traités avec l'erlotinib seul ou en combinaison avec l'ivermectine (A) ou avec l'erlotinib seul ou avec l'ivermectine, en présence de TNFα/IL1β (B). E1= erlotinib, 1µM ; I5= ivermectine, 5µM

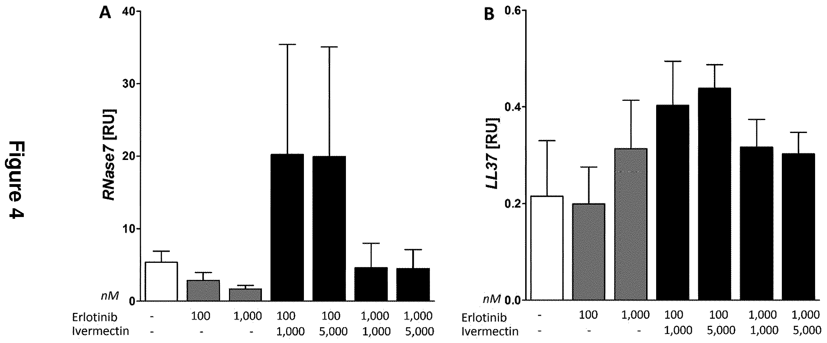

Figure 4. Analyse de l'expression de la RNase7 (A) et du LL37 (B) par les kératinocytes humains primaires traités avec différentes concentrations d'erlotinib seul ou en combinaison avec l'ivermectine à différentes concentrations.

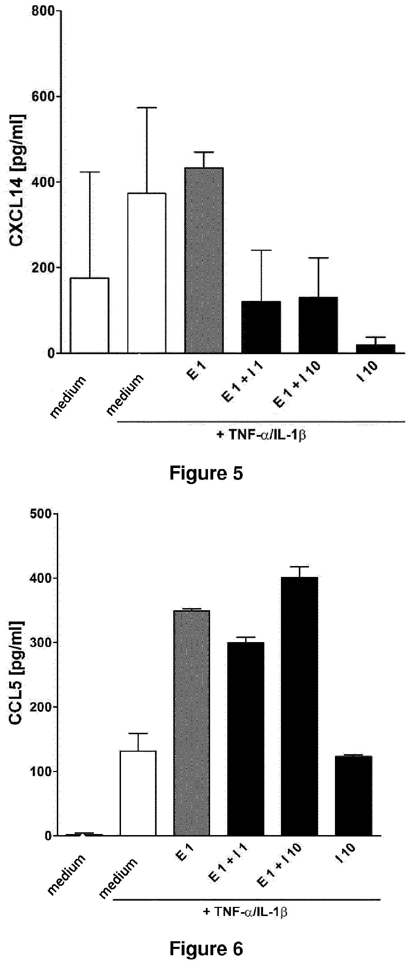

Figure 5. Analyse du niveau protéique de CXCL14 avec le test ELISA chez les kératinocytes humains primaires traités avec l'erlotinib seul ou en combinaison avec l'ivermectine à différentes concentrations. E1= erlotinib, 1µM ; I1= ivermectine, 1µM ; I10= ivermectine, 10µM.
Figure 6. Analyse du niveau protéique de CCL5 avec le test ELISA chez les kératinocytes humains primaires traités avec l'erlotinib seul ou en combinaison avec l'ivermectine à différentes concentrations. E1= erlotinib, 1µM ; I1= ivermectine, 1µM ; I10= ivermectine, 10µM.

### DESCRIPTION DETAILLEE DE L'INVENTION

Les traitements anti-cancéreux et plus particulièrement les thérapies ciblées causent de nombreux effets secondaires notamment au niveau cutané. Parmi les effets toxiques cutanés les plus souvent observés, on retrouve les éruptions papulopustuleuses aussi appelées folliculites. Ces folliculites sont notamment observées chez les patients subissant un traitement chimiothérapeutique et plus particulièrement des thérapies dites ciblées. On peut faire référence notamment aux agents de thérapie ciblée ayant une activité inhibitrice des récepteurs au facteur de croissance épidermique (EGFR) ou encore aux inhibiteurs sélectifs d'une protéine kinase activée par des agents mitogènes telles que les kinases MEK1 et/ou MEK2 (Bragg et al, Dermatology Online Journal, vol. 13, no. 1, 2007,Extrait de l'Internet: URL:http://escholarship.org/uc/item/3426f09x; Gerber et al, Dermatology, vol. 222, no. 2, 1 janvier 2011, pages 144-147).

On entend par folliculites, des éruptions papulopustuleuses observées au niveau des follicules pileux, notamment au niveau des zones séborrhéiques telles que le visage, le cou, le dos, le torse ou encore le cuir chevelu. Elles sont souvent accompagnées de prurit et de lésions érythémateuses mais ne sont jamais associées à des comédons, permettant ainsi de les distinguer clairement de l'acné. On peut distinguer trois différents grades de sévérité des folliculites, le grade 1 correspondant à l'apparition de papules, le grade 2 correspondant à l'apparition de papules et de pustules et enfin le grade 3 correspondant à l'apparition de papules et de pustules accompagnée de prurit et de douleurs cutanées. Les folliculites partagent certaines similitudes avec la rosacée mais s'en distinguent nettement par un certain nombre de caractéristiques. En effet, contrairement à la rosacée, les folliculites induites par des traitements chimiothérapeutiques apparaissent brutalement dès l'introduction du traitement et sont résolues spontanément de façon progressive ou brutale dès l'arrêt du traitement. Par ailleurs, contrairement à la rosacée qui est observée essentiellement au niveau du visage, les folliculites s'étendent généralement à toutes les zones séborrhéiques notamment au niveau du visage, du cuir chevelu, du dos et du tronc.

Les folliculites sont observées chez plus de 85% des patients subissant ces traitements anti-cancéreux et notamment des thérapies ciblées et ce, assez rapidement puisqu'elles apparaissent généralement au bout de 7 à 15 jours. Ces folliculites causées par le traitement anti-cancéreux peuvent également être suivies d'une surinfection par les bactéries *Staphylococcus aureus* aggravant ainsi la sévérité des folliculites observées. La sévérité de ces folliculites varie d'un patient à l'autre. Cependant, il est nécessaire d'interrompre le traitement anti-cancer chez environ 15% des patients présentant ces folliculites.

De récents travaux ont permis de mettre en évidence les mécanismes clés impliqués à l'origine des folliculites induites par des agents anti-cancéreux et notamment par des agents ayant une activité anti-EGFR. Il a en effet été démontré que l'inhibition du récepteur au facteur de croissance épidermique induit l'expression de nombreuses chimiokines proinflammatoires telles que CCL2, CCL5, CCL27 ou encore CXCL14 mais aussi de cytokines telles que celles appartenant à la famille IL-1 à l'origine du recrutement de cellules de l'inflammation (cellules T, cellules de Langerhans, les mastocytes, les macrophages, les granulocytes) au sein de l'aire périfolliculaire générant des papules et pustules folliculaires. Une diminution significative de l'expression de gènes barrière (claudin, occludin) et de peptides antimicrobiens (LL37, HBD3, RNase 7) menant à une sècheresse cutanée et un risque accru d'infections bactériennes, notamment par le *Staphylococcus aureus* (Lichtenberger et al., Sci Transi Med, 2013, 5: 199ra111). Ces mécanismes biologiques se distinguent nettement de ceux impliqués chez les patients atteints de rosacée pour lesquels une activation de cellules T a été mise en évidence associée à la production des cytokines IL17 et IL22 permettant l'attraction de chimiokines spécifiques telles que CXCL1, CXCL8, CCL20 et CCL2 (Gerber et al, Journal of Investigative Dermatology Symposium Proceedings 15, 40-47; 2011).

Tel que précisé précédemment, les agents anti-cancéreux induisant fréquemment des folliculites sont notamment ceux utilisés pour les thérapies dites ciblées. A titre d'exemple, on peut faire référence aux agents ayant une activité anti-EGFR mais aussi les agents inhibiteurs sélectifs d'une protéine kinase activée par des agents mitogènes telles que les kinases MEK1 et/ou MEK2.

Par « agents anti-cancéreux » on entend au sens de la présente invention toute substance chimique ayant pour objectif de traiter le cancer, on englobe par conséquent dans cette définition les «agents chimiothérapeutiques » dits classiques ainsi que les « agents de thérapie ciblée ».

Par « agents chimiothérapeutiques » on entend au sens de la présente invention toute substance chimique ayant pour objectif de traiter le cancer par une action générale visant à affecter la division des cellules, notamment en interagissant avec les structures essentielles de la division et de la survie cellulaires.

Par « agents de thérapie ciblée» on entend au sens de la présente invention, toute substance chimique ayant pour objectif de traiter le cancer en ciblant spécifiquement un récepteur, un gène ou une protéine impliqué dans la ou les voies de signalisation intracellulaires jouant un rôle dans la transformation des cellules normales en cellules cancéreuses ou dans le développement des tumeurs malignes. Plus précisément les agents de thérapie ciblée peuvent être des anticorps monoclonaux ciblant un récepteur cellulaire spécifique ou encore une molécule apte à se fixer à l'un de ces récepteurs ou encore de petites molécules inhibitrices de tyrosine kinase capables d'inhiber les voies de signalisation cellulaires.

Par « anticorps monoclonaux » on entend au sens de la présente invention, un anticorps spécifiquement dirigé contre une « cible » pouvant par exemple être la partie extracellulaire d'un récepteur à la surface des cellules. On fait notamment référence au bevacizumab ciblant le facteur de croissance des vaisseaux endothéliaux (VEGF), ou encore au cetuximab ciblant les récepteurs cellulaires au facteur de croissance épidermique (EGF), ou encore au panitumumab ayant le même mode d'action que le cetuximab et indiqué en l'absence de mutation K-Ras. On peut également faire référence au brentuximab, catumaxomab, ibritumomab, ipiimumab, ofatumumab, rituximab, ou encore au trastuzumab.

Par « petites molécules inhibitrices de tyrosine kinase » au sens de la présente invention on fait notamment référence aux agents capables de pénétrer à l'intérieur des cellules cancéreuses et d'affecter les voies de signalisation cellulaires impliquées dans la croissance cellulaire maligne. Au sens de la présente invention on peut citer l'axitinib, crizotinib, dasatinib, erlotinib, géfitinib, imatinib, lapatinib, nilotinib, ruxolitinib, sorafénib, sunitinib, vandétanib, vémurafénib, evérolimus, régorafénib ou encore le temisrolimus.

Par « agents ayant une activité anti-EGFR » au sens de la présente invention on fait notamment référence aux familles de agents inhibant la voie de transduction du facteur de croissance épidermique EGF. Lesdits agents sont notamment des petites molécules inhibitrices de kinases et/ou encore des anticorps monoclonaux inhibiteurs d'EGFR. Plus précisément on fait référence au cetuximab, au gefitinib, à l'erlotinib, au panitumumab et/ou au lapatinib.

Par « agents inhibiteurs sélectifs d'une protéine kinase activée par des agents mitogènes » aussi nommés « inhibiteurs MEK » au sein de la présente invention, on fait référence aux agents inhibiteurs sélectifs d'une protéine kinase activée par des agents mitogènes telles que les kinases MEK1 et/ou MEK2 par exemple le trametinib, selumetinib, binimetinib, cobimetinib.

De façon surprenante la Demanderesse a pu mettre en évidence qu'un composé de la famille des avermectines, préférentiellement l'ivermectine pour leur utilisation par voie topique permet de traiter et/ou de prévenir de façon efficace les folliculites induites par des agents anti-cancéreux.

L'objet de la présente invention porte donc sur un composé de la famille des avermectines, préférentiellement l'ivermectine pour son utilisation dans le traitement et/ou la prévention des folliculites causées par des agents anti-cancéreux ayant une activité anti-EGFR.

Selon un autre aspect, la présente invention porte également sur un composé de la famille des avermectines, préférentiellement l'ivermectine pour son utilisation dans le traitement et/ou la prévention des folliculites causées par des agents anti-cancéreux ayant une activité anti-EGFR ainsi que leur potentielle aggravation en présence de bactéries telles que les *Staphylococcus aureus.*

Au sens de la présente invention, les agents anti-cancéreux à l'origine des folliculites sont ceux ayant une activité anti-EGFR tels que définis précédemment.

La présente invention porte également sur une composition pharmaceutique comprenant un composé de la famille des avermectines, préférentiellement l'ivermectine en présence d'un support pharmaceutiquement acceptable pour son utilisation dans le traitement et/ou la prévention des folliculites causées par des agents anti-cancéreux ayant une activité anti-EGFR.

Selon un autre aspect, la présente invention porte également sur une composition pharmaceutique comprenant un composé de la famille des avermectines, préférentiellement l'ivermectine en présence d'un support pharmaceutiquement acceptable pour son utilisation dans le traitement et/ou la prévention des folliculites causées par des agents anti-cancéreux ayant une activité anti-EGFR, ainsi que leur potentielle aggravation en présence de bactéries telles que les *Staphylococcus aureus.*

Selon l'invention, la composition comprenant un composé de la famille des avermectines, préférentiellement l'ivermectine, est sous forme adaptée à une administration par voie topique, dans une quantité thérapeutique efficace chez un patient souffrant ou pouvant souffrir de folliculites induites par des agents anti-cancéreux ayant une activité anti-EGFR.

Selon un mode de réalisation, la présente invention consiste à administrer par voie topique une composition comprenant un composé de la famille des avermectines, préférentiellement l'ivermectine, dans une quantité efficace simultanément à l'administration de l'agent anti-cancéreux. La composition comprenant un composé de la famille des avermectines de préférence l'ivermectine et les agents anti-cancéreux ayant une activité anti-EGFR peuvent être administrés simultanément par une même voie d'administration ou par deux voies d'administration différentes. De préférence la composition selon la présente invention est administrée par voie topique.

La famille des avermectines est un groupe de lactones macrocycliques produit par la bactérie *Streptomyces avermitilis* (Reynolds JEF (Ed) (1993) Martindale. The extra pharmacopoeia. 29th Edition. Pharmaceutical Press, London). Parmi ces lactones macrocycliques appartenant à la classe des avermectines, on peut citer l'ivermectine, l'avermectine, l'abamectine, la doramectine, l'éprinomectine, la selamectine, l'aversectine B, AB ou C, l'émamectine B1b et leurs dérivés, ou la latidectine. Selon l'invention, le composé de la famille des avermectines est préférentiellement l'ivermectine.

L'ivermectine est un mélange de 22,23-dihydroavermectine B₁ₐ et 22,23-dihydroavermectine B_{1b}. L'ivermectine contient majoritairement du 22,23-dihydroavermectine B₁ₐ.

L'ivermectine est connue dans l'art antérieur pour ses propriétés antiparasitaires, notamment contre Demodex, et anthelminthiques. Au milieu des années 80, la molécule a été présentée comme médicament antiparasitaire de large spectre à usage vétérinaire (CAMPBELL, W.C., et al., (1983). Ivermectin: a patent new antiparasitic agent. Science, 221, 823-828.). Elle est efficace contre la plupart des vers intestinaux communs (excepté les ténias), la plupart des acarides, et quelques poux. Elle présente une affinité importante pour les canaux chlorure glutamate-dépendants, notamment ceux dépendant du neuromédiateur GABA (acide gamma-amino-butyrique), présents dans les cellules nerveuses et musculaires des invertébrés, lui conférant une activité antiparasitaire. Plus particulièrement, sa fixation sur ces canaux favorise une augmentation de la perméabilité membranaire aux ions chlorures entraînant une hyperpolarisation de la cellule nerveuse ou musculaire. Il en résulte une paralysie neuromusculaire pouvant entraîner la mort de certains parasites. L'ivermectine interagit également avec d'autres canaux chlore.

L'ivermectine a aussi été utilisée dans le traitement de l'acné vulgaire. Le brevet US 6,399,652 décrit l'utilisation de l'ivermectine dans le traitement de l'acné en complément d'une autre composition contenant un autre actif qui peut être le peroxyde de benzoyle, le résorcinol, l'acide salicylique, un opioïde, la trétinoïne, un antibiotique ou l'isotrétinoïne.

L'ivermectine permet également de réduire et d'éliminer le parasite Demodex pour lequel une augmentation de sa densité a été observée chez des patients souffrant d'acné (Zhao et al., J Zhejiang Univ Sci B., 2012, 13, 192-202) et est également efficace dans le traitement de la rosacée (Stein et al, Journal of Drugs in Dermatology vol. 13, no. 3, 1 mars 2014, pages 316-323; US 2015/011489 A1; US 5 952 372 A).

Sans vouloir être lié par une quelconque explication théorique sur le mode d'action et l'efficacité des composés, l'invention tire profit des propriétés anti-inflammatoires des composés de la famille des avermectines ainsi qu'à leurs propriétés antiparasitaires notamment au niveau de Demodex. En effet, les composés de la famille des avermectines sont des agents anti-inflammatoires efficaces, capables de bloquer certains médiateurs de l'inflammation et ainsi de diminuer les symptômes causés par l'inflammation.

Dans un mode de réalisation particulier, le composé de la famille des avermectines, préférentiellement l'ivermectine est présent dans la composition entre 0,001 et 10 %, préférentiellement entre 0,001 et 8%, de préférence entre 0,001 et 5 %, encore plus préférentiellement entre 0,1 et 3%, de préférence encore entre 0,1 et 2%, et d'une manière encore plus préférée 1 % en poids, par rapport au poids total de la composition.

Dans un mode de réalisation préféré, la composition selon la présente invention comprenant le composé de la famille des avermectines, préférentiellement l'ivermectine est administrée simultanément aux agents anti-cancéreux. La composition selon l'invention et les agents anti-cancéreux peuvent être administrés par la même voie d'administration ou par deux voies d'administration différentes.

Par voie topique, on entend une administration sur la peau, les phanères ou les muqueuses. Plus précisément, les compositions pharmaceutiques à base de composés selon l'invention sont destinées au traitement de la peau et plus préférentiellement au niveau des zones riches en follicules pileux.

Les compositions utiles selon l'invention peuvent se présenter sous forme d'émulsions, d'onguents, de crèmes, de laits, de lotions, de pommades, de poudres, de tampons imbibés, de solutions, de gels, de sprays, de lotions, de suspensions, de savon ou de shampoings. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique. La présente invention vise plus particulièrement une composition pharmaceutique contenant, dans un support pharmaceutiquement acceptable, au moins un composé de la famille des avermectines tel que défini ci-dessus. De préférence, le composé de la famille des avermectines est l'ivermectine.

Dans les compositions utiles selon l'invention, le composé de la famille des avermectines et de préférence l'ivermectine, peut être combiné avec d'autres composés pharmaceutiques ou cosmétiques, seuls ou en mélange, tels des rétinoïdes, corticosteroïdes, des agents anti-inflammatoires non stéroïdiens, des antibiotiques ou agents antifongiques. Les compositions pharmaceutiques selon l'invention peuvent, en outre, contenir des additifs inertes ou des combinaisons de ces additifs et notamment: des agents mouillants, des agents dépigmentants, des émollients, des agents hydratants, des agents stabilisants, des agents régulateurs d'humidité, des agents régulateurs de pH, des agents modificateurs de pression osmotique, des agents émulsionnants, des filtres UV-A et UV-B, des antioxydants.

Selon un mode de réalisation préféré, la composition comprend un ou plusieurs composés de la famille des avermectines, un ou plusieurs solvants, une phase grasse, un ou plusieurs tensioactifs et de l'eau.

L'homme du métier veillera à choisir les additifs et excipients constituant les compositions selon l'invention en fonction de la forme galénique souhaitée et de manière à ce que les propriétés avantageuses de la composition selon l'invention soient respectées.

Selon un mode de réalisation préféré la composition pharmaceutique selon la présente invention, utilisée pour traiter et/ou prévenir les folliculites causées par les agents anti-cancéreux ayant une activité anti-EGFR est administrée en même temps que lesdits agents anti-cancéreux.

Selon un autre mode de réalisation, la présente invention porte aussi sur la composition pharmaceutique selon la présente invention, pour une utilisation dans le traitement et/ou la prévention de l'aggravation desdites folliculites, par la prolifération de pathogènes cutanés tels que le *Staphylococcus aureus.*

Dans un mode de réalisation, le terme " traitement " ou " traiter " désigne une amélioration, la prophylaxie, ou l'inversion de la folliculite, ou au moins d'un symptôme associé à la folliculite. Dans un autre mode de réalisation, " traitement " ou " traiter " désigne une amélioration, la prophylaxie, ou l'inversion d'au moins un paramètre physique mesurable associé à la folliculite, qui n'est pas nécessairement discernable chez ou par le sujet traité. Dans un autre mode de réalisation supplémentaire, " traitement " ou " traiter " désigne l'inhibition ou le ralentissement de la progression de la folliculite, physiquement, par exemple, la stabilisation d'un symptôme discernable, physiologiquement, par exemple, la stabilisation d'un paramètre physique, ou les deux. Dans un autre mode de réalisation, " traitement " ou " traiter " désigne le retard de l'apparition de la folliculite ou d'un symptôme qui lui est associé.

Dans le présent contexte, "prévention" ou " prévenir " désigne une réduction du risque d'acquisition de la folliculite mais aussi une réduction, une inhibition ou un ralentissement de l'apparition des symptômes liés aux folliculites. Des symptômes caractéristiques des folliculites sont par exemple, des papules ou encore des pustules au niveau des follicules pileux, des érythèmes, des pustules, des lésions érythémateuses ou encore du prurit apparaissant principalement sur le visage, le cou, le cuir chevelu, le dos et le thorax, *i.e.* les régions cutanées où on trouve le plus grand nombre de glandes sébacées.

Au sens de la présente invention, par « patient » on entend tout mammifère, et plus particulièrement les êtres humains, hommes ou femmes.

La quantité réellement administrée de composé de la famille des avermectines, préférentiellement d'ivermectine, à mettre en oeuvre selon l'invention dépend de l'effet thérapeutique ou préventif recherché, et peut donc varier dans une large mesure. L'homme de l'art, en particulier le médecin peut aisément, sur la base de ses connaissances générales déterminer les quantités appropriées.

Ainsi, et selon une forme de réalisation préférée, la composition est administrée 1 à 2 fois/jour pour un effet thérapeutique. De préférence, le traitement peut avoir une durée allant de 1 semaine à 6 mois, renouvelable, et de préférence de 2 semaines à 4 mois. Les cures peuvent être renouvelées en cycle avec ou sans période de repos. L'administration de la composition selon l'invention pouvant ou pas être simultanée à l'administration des agents anti-cancéreux.

Ainsi, et selon une autre forme de réalisation préférée, la composition est administrée 1 à 2 fois/jour et simultanément aux agents anti-cancéreux pour un effet préventif. De préférence, le traitement peut avoir une durée allant de 1 semaine à 6 mois, renouvelable, et de préférence de 2 semaines à 4 mois. Les cures peuvent être renouvelées en cycle avec ou sans période de repos.

Selon un autre mode de réalisation préférée, la composition selon la présente invention est administrée simultanément aux agents anti-cancéreux. La composition comprenant un composé de la famille des avermectines de préférence l'ivermectine et les agents anti-cancéreux peuvent être administrés simultanément ou séquentiellement. De préférence selon la présente invention la composition comprenant un composé de la famille des avermectines de préférence l'ivermectine et les agents anti-cancéreux sont administrés simultanément.

L'administration de ladite composition et des agents anti-cancéreux peut se faire par une même voie d'administration ou par deux voies d'administration différentes. De préférence la composition selon la présente invention est administrée par voie topique.

Dans le contexte de l'invention, on entend par « dose thérapeutique efficace » ou « quantité thérapeutique efficace » la dose, la quantité thérapeutique qui empêche, supprime ou réduit les effets délétères des folliculites traitées chez le patient. Il est entendu que la dose administrée peut être adaptée par l'homme du métier en fonction du patient, de la sévérité des folliculites, du mode d'administration, etc.

L'exemple et les figures illustrent l'invention sans en limiter la portée.

### EXEMPLE

Une étude a été réalisée sur des kératinocytes primaires humains *in vitro,* afin de déterminer l'effet de l'ivermectine sur la régulation des gènes dont l'expression a été identifiée comme étant modulée par les inhibiteurs EGFR. Lesdits gènes identifiés sont les chimiokines pro-inflammatoires CCL2, CCL5, CCL27, CXCL14 et les cytokines de la famille IL-1β mais également les gènes de peptides anti-microbiens RNase7, HBD3 et LL37.

Un autre objectif de la présente étude était d'identifier l'effet de l'ivermectine sur le niveau protéique de CXCL14 et de CCL5.

Pour ce faire, des kératinocytes primaires humains ont été cultivés avec différentes concentrations d'inhibiteur d'EGFR (ici l'erlotinib) chacune avec +/- TNF-α/IL-1β, différentes concentrations d'ivermectine seule chacune avec +/- TNF-α/IL-1β ou avec différentes concentrations d'erlotinib et d'ivermectine chacune avec +/- TNF-α/IL-1β. L'addition des cytokines pro-inflammatoires TNFα et IL1β permet de mimer l'inflammation cutanée.

Les différentes concentrations utilisées étaient les suivantes:

| | |
|---|---|
| Erlotinib: | 0 - 0.1 µM - 1 µM - 10 µM (each with +/- TNF-α/IL-1β) |
| Ivermectine: | 0 - 0.1 µM - 1 µM - 10 µM (each with +/- TNF-α/IL-1β) |

Co-stimulation:
Erlotinib (0.1 µM) + Ivermectin (1 µM) (+/- TNF-α/IL-1β)
Erlotinib (0.1 µM) + Ivermectin (5 µM) (+/- TNF-α/IL-1β)
Erlotinib (1 µM) + Ivermectin (1 µM) +/- TNF-α/IL-1β)
Erlotinib (1 µM) + Ivermectin (5 µM) (+/- TNF-α/IL-1β)

L'expression des gènes CXCL14, CCL5 (chimiokines), RNase7, HBD3, LL37 (peptides antimicrobiens) a été analysée par PCR quantitative. La concentration protéique des chimiokines CXCL14 et CCL5 a été quantifiée à l'aide d'un test ELISA.

### Résultats:

Le traitement des kératinocytes primaires humains avec l'erlotinib seul ou en présence d'ivermectine +/- TNFα/IL1β a permis de mettre en évidence une nette augmentation du niveau d'expression des gènes CCL5 et HBD3 en présence de TNFα/IL1β et une altération significative de l'induction par l'erlotinib de l'expression de CXCL14 et CCL5 en présence d'ivermectine.

Ces tests ont montré la capacité de l'ivermectine à réduire la surexpression de CCL5 et CXCL14 induite par l'erlotinib mais également à induire l'expression de HBD3 diminuée en présence d'erlotinib.

L'ivermectine permet également de rétablir l'expression de RNase 7 fortement diminuée en présence d'erlotinib mais aussi d'induire une forte expression de celle-ci. Dans une moindre mesure, les mêmes effets ont été observés pour LL37.

Ces essais ont également permis de mettre en évidence une augmentation de la concentration protéique de CXCL14 et CCL5 en présence d'erlotinib et de TNFα/IL1β et la capacité de l'ivermectine de diminuer le niveau protéique de CXCL14 et dans une moindre mesure de CCL5.

L'ensemble de ces résultats met donc en évidence la capacité de l'ivermectine à modifier rétablir le profil d'expression de base des gènes modulés par les inhibiteurs EGFR, ce qui conforte son intérêt pour traiter et/ou prévenir les folliculites induites par les inhibiteurs EGFR mais aussi leur surinfection par les *Staphylococcus aureus.*

## Revendications

1. Composé de la famille des avermectines pour une utilisation dans le traitement et/ou la prévention d'une folliculite causée par un agent anti-cancéreux ayant une activité anti-EGFR.

2. Composé de la famille des avermectines pour une utilisation selon la revendication 1, où le composé de la famille des avermectines est choisi parmi le groupe consistant en l'ivermectine, l'avermectine, l'abamectine, la doramectine, l'éprinomectine, la selamectine, l'aversectine B, AB ou C, l'émamectine B1b ou la latidectine.

3. Composé de la famille des avermectines pour une utilisation selon la revendication 1 ou 2, où le composé de la famille des avermectines est l'ivermectine.

4. Composé de la famille des avermectines pour une utilisation selon la revendication 1, où l'agent thérapeutique ayant une activité anti-EGFR est choisi parmi le cetuximab, le gefitinib, l'erlotinib, le panitumumab et/ou le lapatinib.

5. Composé de la famille des avermectines pour une utilisation selon la revendication 1 où les folliculites induites par un agent anti-cancéreux ayant une activité anti-EGFR sont des folliculites aggravées par la prolifération d'un pathogène cutané.

6. Composé de la famille des avermectines pour une utilisation selon la revendication 5, où ledit pathogène cutané est *Staphylococcus aureus.*

7. Composé de la famille des avermectines pour une utilisation selon l'une des revendications précédentes, où le composé est sous forme de composition pharmaceutique.

8. Composé de la famille des avermectines pour une utilisation selon la revendication 7, où la composition est sous une forme adaptée à une administration par voie topique.

9. Composé de la famille des avermectines pour une utilisation selon la revendication 8, où la composition est sous forme d'émulsion, de crème, de lotion, de gel, de solution, ou d'onguent.

10. Composé de la famille des avermectines pour une utilisation selon l'une des revendication 7 à 9, où la composition comprend entre 0.001 et 10%, de préférence entre 0.001 et 8%, de façon plus préférée entre 0.001 et 5% et de façon encore plus préférée entre 0.1 et 3% en poids de composé de la famille des avermectines par rapport au poids total de la composition.

11. Composé de la famille des avermectines pour une utilisation selon la revendication 10, où la composition comprend 1% en poids de composé de la famille des avermectines en comparaison au poids total de la composition.

## Patentansprüche

1. Verbindung aus der Familie der Avermectine für eine Verwendung bei der Behandlung und/oder Prävention einer Follikulitis, die durch einen Anti-Krebs-Wirkstoff mit einer Anti-EGFR-Wirkung verursacht wird.

2. Verbindung aus der Familie der Avermectine für eine Verwendung nach Anspruch 1, wobei die Verbindung aus der Familie der Avermectine in der Gruppe ausgewählt ist, umfassend Ivermectin, Avermectin, Abamectin, Doramectin, Eprinomectin, Selamectin, Aversectin B, AB oder C, Emamectin B1b oder Latidectin.

3. Verbindung aus der Familie der Avermectine für eine Verwendung nach Anspruch 1 oder 2, wobei die Verbindung aus der Familie der Avermectine das Ivermectin ist.

4. Verbindung aus der Familie der Avermectine für eine Verwendung nach Anspruch 1, wobei der therapeutische Wirkstoff, der eine Anti-EGFR-Aktivität hat, unter Cetuximab, Gefitinib, Erlotinib, Panitumumab und/oder Lapatinib ausgewählt ist.

5. Verbindung aus der Familie der Avermectine für eine Verwendung nach Anspruch 1, wobei die Follikulitiden, die durch einen Anti-Krebs-Wirkstoff mit einer Anti-EGFR-Aktivität induziert werden, Follikulitiden sind, die durch die Vermehrung eines Hauterregers verschlimmert werden.

6. Verbindung aus der Familie der Avermectine für eine Verwendung nach Anspruch 5, wobei der Hauterreger *Staphylococcus aureus* ist.

7. Verbindung aus der Familie der Avermectine für eine Verwendung nach einem der vorhergehenden Ansprüche, wobei die Verbindung in Form einer pharmazeutischen Zusammensetzung vorhanden ist.

8. Verbindung aus der Familie der Avermectine für eine Verwendung nach Anspruch 7, wobei die Zusammensetzung in einer Form vorhanden ist, die für eine Verabreichung auf topischem Weg geeignet ist.

9. Verbindung aus der Familie der Avermectine für eine Verwendung nach Anspruch 8, wobei die Zusammensetzung in Form einer Emulsion, einer Crème, einer Lotion, eines Gels, einer Lösung oder einer Salbe vorhanden ist.

10. Verbindung aus der Familie der Avermectine für eine Verwendung nach einem der Ansprüche 7 bis 9, wobei die Zusammensetzung zwischen 0,001 und 10 Gew.-%, vorzugsweise zwischen 0,001 und 8 Gew.-%, bevorzugter zwischen 0,001 und 5 Gew.-% und noch bevorzugter zwischen 0,1 und 3 Gew.-% einer Verbindung aus der Familie der Avermectine bezogen auf das Gesamtgewicht der Zusammensetzung umfasst.

11. Verbindung aus der Familie der Avermectine für eine Verwendung nach Anspruch 10, wobei die Zusammensetzung 1 Gew.-% einer Verbindung aus der Familie der Avermectine verglichen mit dem Gesamtgewicht der Zusammensetzung umfasst.

## Claims

1. Compound from the avermectin family for use in the treatment and/or prevention of folliculitis caused by an anti-cancer agent having anti-EGFR activity.

2. Compound from the avermectin family for use according to claim 1, wherein the compound from the avermectin family is selected from the group consisting of ivermectin, avermectin, abamectin, doramectin, eprinomectin, selamectin, aversectin B, AB or C, emamectin B1b or latidectin.

3. Compound from the avermectin family for use according to claim 1 or 2, wherein the compound from the avermectin family is ivermectin.

4. Compound from the avermectin family for use according to claim 1, wherein the therapeutic agent having anti-EGFR activity is selected from cetuximab, gefitinib, erlotinib, panitumumab and/or lapatinib.

5. Compound from the avermectin family for use according to claim 1 wherein the folliculitis induced by an anti-cancer agent having anti-EGFR activity are folliculitis aggravated by the proliferation of a skin pathogen.

6. Compound from the avermectin family for use according to claim 5, wherein said skin pathogen is *Staphylococcus aureus.*

7. Compound from the avermectin family for use according to one of the preceding claims, wherein the compound is in the form of a pharmaceutical composition.

8. Compound from the avermectin family for use according to claim 7, wherein the composition is in a form suitable for topical administration.

9. Compound from the avermectin family for use according to claim 8, wherein the composition is the form of an emulsion, cream, lotion, gel, solution, or ointment.

10. Compound from the avermectin family for use according to one of claims 7 to 9, wherein the composition includes between 0.001 and 10%, preferably between 0.001 and 8%, more preferably between 0.001 and 5% and even more preferably between 0.1 and 3% by weight of the compound from the avermectin family relative to the total weight of the composition.

11. Compound from the avermectin family for use according to claim 10, wherein the composition includes 1% by weight of the compound from the avermectin family in comparison to the total weight of the composition.
